(19) Europäisches Patentamt
European Patent Office
Office européen des brevets

(11) **EP 4 407 554 A1**

(12) **EUROPEAN PATENT APPLICATION**
published in accordance with Art. 153(4) EPC

(43) Date of publication:
**31.07.2024 Bulletin 2024/31**

(21) Application number: **22872719.4**

(22) Date of filing: **08.09.2022**

(51) International Patent Classification (IPC):
**G06T 7/00** (2017.01)        **A61B 6/00** (2024.01)
**A61B 6/03** (2006.01)

(52) Cooperative Patent Classification (CPC):
**A61B 6/00; A61B 6/03; G06T 7/00**

(86) International application number:
**PCT/JP2022/033653**

(87) International publication number:
**WO 2023/047963 (30.03.2023 Gazette 2023/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **21.09.2021 JP 2021153427**

(71) Applicant: **Teikyo University
Tokyo 173-8605 (JP)**

(72) Inventors:
• **KOTOKU Junichi
  Tokyo 173-8605 (JP)**
• **TSUJI Takumasa
  Tokyo 173-8605 (JP)**

(74) Representative: **Cabinet Chaillot
16/20, avenue de l'Agent Sarre
B.P. 74
92703 Colombes Cedex (FR)**

(54) **MEDICAL IMAGE DIAGNOSTICS ASSISTANCE DEVICE, MEDICAL IMAGE DIAGNOSTICS ASSISTANCE METHOD, AND PROGRAM**

(57) A medical image diagnostics assistance device (1) for assisting diagnosis of a medical image includes a classification model (1A) for classifying at least presence or absence of a disease from the medical image, a prediction unit (11) that carries out prediction using the classification model (1A), and a learning unit (12) that carries out supervised learning of the classification model (1A). In the supervised learning, a training medical image for which at least the presence or absence of the disease is previously known is used as supervised data. The classification model (1A) is constructed by a convolutional neural network (1A1) and an attention branch network (1A2) that visualizes an interest region of the convolutional neural network (1A1). In a stage where the supervised learning is carried out, the attention branch network (1A2) is provided with preliminary information indicating a classification region which is a region required for classifying the presence or absence of the disease on the training medical image.

FIG. 1

**Description**

[Technical Field]

The present invention relates to a medical image diagnostics assistance device, a medical image diagnostics assistance method, and a program.
Priority is claimed on Japanese Patent Application No. 2021-153427, filed on September 21, 2021, the content of which is incorporated herein by reference.

[Background Art]

In the related art, an image diagnostics assistance device that assists a doctor to carry out image diagnosis is known (for example, refer to Patent Document 1). In a technique disclosed in Patent Document 1, diagnostic ability of an endoscope image belonging to a convolutional neural network (CNN) is used. Specifically, according to the technique disclosed in Patent Document 1, a lesion estimation unit uses the convolutional neural network to estimate a lesion name and a lesion location of a lesion existing inside the endoscopic image represented by endoscopic image data, and accuracy in the lesion name and the lesion location. In addition, according to the technique disclosed in Patent Document 1, a learning process of the convolutional neural network is carried out by using reference data (supervised data) subjected to marking processing in advance by an experienced endoscopist.
Incidentally, in a medical field, it is necessary to consider a black box problem of artificial intelligence (AI). That is, it is necessary to consider the black box problem of image diagnostics assistance AI such as the image diagnostics assistance device disclosed in
Patent Document 1.

[Citation List]

[Patent Document]

[Patent Document 1]:
Japanese Patent No. 6657480

[Summary of Invention]

[Technical Problem]

Therefore, through intensive research, the present inventors used a chest X-ray image as a medical image, and adopted the convolutional neural network and an attention branch network (ABN) for visualizing an interest region of the convolutional neural network. In this manner, a classification model configured to classify the presence or absence of a disease from the chest X-ray image was constructed, and the interest region of the convolutional neural network was verified. As a result, the present inventors discovered that the convolutional neural network may focus on a region which is not required for classifying the presence or absence of the disease on the chest X-ray image.
In this way, for the medical AI that focused on the region which is not required for classifying the presence or absence of the disease on the chest X-ray image, the present inventors further advanced the research after asking whether the present invention can achieve reliability from a medical site, for example, such as reliability from medical specialists.

[Solution to Problem]

Specifically, through the intensive research, the present inventors provided the attention branch network with preliminary information indicating a region for classifying the presence or absence of the disease on the chest X-ray image. As a result, the convolutional neural network can be prevented from focusing on the region which is not required for classifying the presence or absence of the disease on the chest X-ray image.
That is, an object of the present invention is to provide a medical image diagnostics assistance device, a medical image diagnostics assistance method, and a program, in which a convolutional neural network can be prevented from focusing on a region which is not required for classifying the presence or absence of a disease on a medical image.
According to an aspect of the present invention, there is provided a medical image diagnostics assistance device for assisting diagnosis of a medical image. The medical image diagnostics assistance device includes a classification model configured to classify at least presence or absence of a disease from the medical image, a prediction unit configured to carry out prediction using the classification model, and a learning unit configured to carry out supervised learning of the classification model before the classification model is used by the prediction unit. In the supervised learning carried out by the learning unit, a training medical image for which at least the presence or absence of the disease is previously known is used as supervised data. The classification model is constructed by a convolutional neural network and an attention branch network that visualizes an interest region of the convolutional neural network. In a stage where the supervised learning of the classification model is carried out by the learning unit, the attention branch network is provided with preliminary information indicating a classification region which is a region required for classifying the presence or absence of the disease on the training medical image.

In the medical image diagnostics assistance device according to the aspect of the present invention, the attention branch network may include a feature extractor configured to generate a feature quantity map by extracting a feature quantity required for classifying the medical image, an attention branch configured to generate an attention map using class activation mapping, and a perception branch. At the stage where the supervised learning of the classification model is carried out by the learning unit, the attention map generated by the attention branch may be reflected in the feature quantity map generated by the feature extractor. The perception branch may output the feature quantity map weighted by the attention map, as a classification result of the training medical image. A loss function of the attention branch network may be a sum of a learning error of the attention branch, a learning error of the perception branch, and a regularization term. The regularization term may be Frobenius norm of a matrix obtained by a Hadamard product of the attention map and a weight map, and the weight map may correspond to the classification region.

In the medical image diagnostics assistance device according to the aspect of the present invention, in the stage where the supervised learning of the classification model is carried out by the learning unit, the attention branch network may receive the weight map prepared by carrying out convex hull processing on a segmentation image of a first portion which is a portion of the classification region.

In the medical image diagnostics assistance device according to the aspect of the present invention, in the stage where the supervised learning of the classification model is carried out by the learning unit, the attention branch network may receive the weight map prepared by combining a segmentation image of a first portion which is a portion of the classification region and a segmentation image of a second portion which is another portion of the classification region.

In the medical image diagnostics assistance device according to the aspect of the present invention, the segmentation image of the first portion and/or a combination of the segmentation image of the first portion and a segmentation image of a second portion which is another portion of the classification region may be generated by using U-Net.

In the medical image diagnostics assistance device according to the aspect of the present invention, any one of VGG16, ResNet50, and DenseNet121 may be used as the convolutional neural network.

In the medical image diagnostics assistance device according to the aspect of the present invention, the attention branch network may include a feature extractor configured to generate a feature quantity map by extracting a feature quantity required for classifying the medical image, an attention branch configured to generate an attention map using class activation mapping, and a perception branch. The attention map generated by the attention branch may be reflected in the feature quantity map generated by the feature extractor. The perception branch may output the feature quantity map weighted by the attention map, as a classification result of the medical image. A loss function of the attention branch network may be a sum of a learning error of the attention branch, a learning error of the perception branch, and a regularization term. The regularization term may be Frobenius norm of a matrix obtained by a Hadamard product of the attention map and a weight map, and the weight map may correspond to the prediction region.

According to another aspect of the present invention, there is provided a medical image diagnostics assistance method for assisting diagnosis of a medical image. The medical image diagnostics assistance method includes a prediction step of carrying out prediction using a classification model configured to classify at least presence or absence of a disease from the medical image, and a learning step of carrying out supervised learning of the classification model before the prediction step is carried out. In the supervised learning carried out in the learning step, a training medical image for which at least the presence or absence of the disease is previously known is used as supervised data. The classification model is constructed by a convolutional neural network and an attention branch network that visualizes an interest region of the convolutional neural network. In the learning step, the attention branch network is provided with preliminary information indicating a classification region which is a region required for classifying the presence or absence of the disease on the training medical image.

According to still another aspect of the present invention, there is provided a program that causes a computer to carry out steps including a prediction step of carrying out prediction using a classification model configured to classify at least presence or absence of a disease from the medical image, and a learning step of carrying out supervised learning of the classification model before the prediction step is carried out. In the supervised learning carried out in the learning step, a training medical image for which at least the presence or absence of the disease is previously known is used as supervised data. The classification model is constructed by a convolutional neural network and an attention branch network that visualizes an interest region of the convolutional neural network. In the learning step, the attention branch network is provided with preliminary information indicating a classification region which is a region required for classifying the presence or absence of the disease on the training medical image.

[Advantageous Effects of Invention]

According to the present invention, it is possible to provide a medical image diagnostics assistance device, a medical image diagnostics assistance method, and a program, in which a convolutional neural network can be prevented from focusing on a region which is not required for classifying the presence or absence of a disease on a medical image.

[Brief Description of Drawings]

FIG. 1 is a diagram showing an example of a configuration of a medical image diagnostics assistance device according to a first embodiment.
FIG. 2 is a diagram showing an example of a configuration of an attention branch network represented in FIG. 1.
FIG. 3 is a diagram showing a method for preparing a weight map in A first example of the medical image diagnostics assistance device according to the first embodiment.
FIG. 4 is a diagram showing a method for preparing a weight map in a second example of the medical image diagnostics assistance device of the first embodiment.
FIG. 5 is a diagram showing a calculation process of a regularization term in the medical image diagnostics assistance device of the first embodiment.
FIG. 6 is a diagram showing an interest region of a convolutional neural network of the medical image diagnostics assistance device of the first embodiment.
FIG. 7 is a flowchart showing an example of a process carried out in the medical image diagnostics assistance device of the first embodiment.
FIG. 8 is a diagram showing an interest region of a convolutional neural network of a medical image diagnostics assistance device according to a second embodiment.

[Description in Embodiments]

Hereinafter, embodiments of a medical image diagnostics assistance device, a medical image diagnostics assistance method, and a program of the present invention will be described with reference to the drawings.

[First Embodiment]

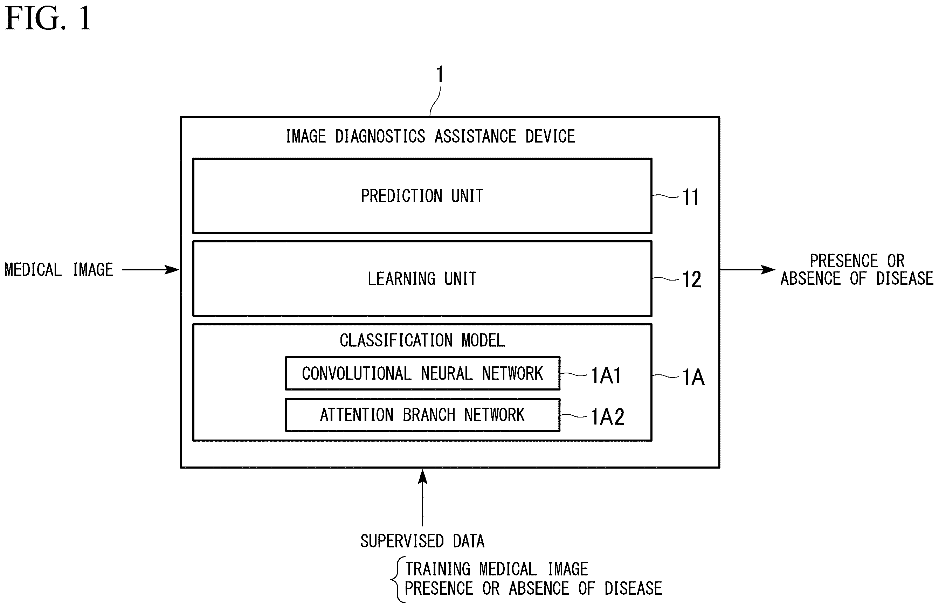

FIG. 1 is a diagram showing an example of a configuration of a medical image diagnostics assistance device 1 of a first embodiment.
In the example indicated in FIG. 1, for example, the medical image diagnostics assistance device 1 of the first embodiment assists medical specialists to carry out diagnosis of a medical image. For example, the medical image as a diagnostics assistance target of the medical image diagnostics assistance device 1 includes an X-ray image such as a chest X-ray image, an echo image, a magnetic resonance imaging (MRI) image, a computed tomography (CT) image, and a fundus image.
The medical image diagnostics assistance device 1 includes a prediction unit 11, a learning unit 12, and a classification model 1A. The classification model 1A classifies presence or absence of a disease from the medical image. That is, the classification model 1A classifies the medical image which needs diagnosis to determine whether or not there is a disease into the medical image having the disease and the medical image having no disease. The prediction unit 11 carries out prediction using the classification model 1A. The learning unit 12 carries out supervised learning of the classification model 1A before the classification model 1A is used by the prediction unit 11. In the supervised learning carried out by the learning unit 12, a training medical image for which the presence or absence of the disease is previously known is used as supervised data.
In the example indicated in FIG. 1, in a learning stage where the learning unit 12 carries out supervised learning of the classification model 1A, a training medical image in which the presence or absence of a disease is known is input to the medical image diagnostics assistance device 1, as the supervised data. That is, the supervised data used for the supervised learning of the classification model 1A is a set of the training medical image and information indicating whether or not an imaging subject of the training medical image has the disease inside the training medical image.
In a prediction stage where the prediction unit 11 predicts the presence or absence of the disease from the medical image by using the classification model 1A, the medical image as a classification target of the classification model 1A is input to the medical image diagnostics assistance device 1, and the classification model 1A classifies the input medical images into the medical image having the disease and the medical image having no disease. Furthermore, a classification result obtained by the classification model 1A (that is, information indicating whether each medical image is an image having the disease or an image having no disease) is output from the medical image diagnostics assistance device 1.

As described above, in a medical field, it is necessary to consider a black box problem of AI. Specifically, unless medical specialists using medical AI cannot recognize which region of the medical image the medical AI focuses on, there is a possibility that the medical specialists do not reliably use the medical AI.

Therefore, in the example indicated in FIG. 1, the classification model 1A is constructed by a convolutional neural network 1A1 and an attention branch network 1A2 that visualizes an interest region of the convolutional neural network 1A1. The attention branch network 1A2 is a classification model in which visualization of the interest region of the convolutional neural network 1A1 using visual explanation and improvement in prediction accuracy are simultaneously realized. For example, details of the attention branch network 1A2 are described in the following document.

H. Fukui, T. Hirakawa, T. Yamashita, and H. Fujiyoshi, "Attention branch network: Learning of attention mechanism for visual explanation," Proc. IEEE Comput. Soc. Conf. Comput. Vis. Pattern Recognit., vol. 2019-June, pp. 10697-10706, 2019, doi: 10. 1 109/CVPR.2019.01096.

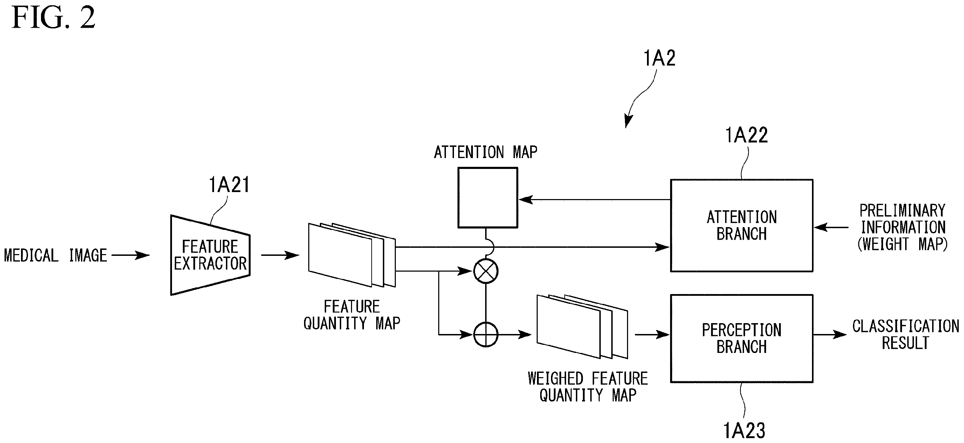

FIG. 2 is a diagram showing an example of a configuration of the attention branch network 1A2 represented in FIG. 1.

In the example indicated in FIG. 2, the attention branch network 1A2 includes a feature extractor 1A21, an attention branch 1A22, and a perception branch 1A23.

For example, in a stage where the supervised learning of the classification model 1A is carried out by the learning unit 12, the feature extractor 1A21 extracts a feature quantity required for classifying the training medical image from the training medical image input to the attention branch network 1A2, and generates a feature quantity map.

The attention branch 1A22 generates an attention map using class activation mapping (CAM) from the feature quantity map generated by the feature extractor 1A21. In the attention branch network 1A2, the attention map generated by the attention branch 1A22 is reflected in the feature quantity map generated by the feature extractor 1A21 (that is, the feature quantity map is weighted by the attention map).

The perception branch 1A23 classifies the feature quantity map weighted by the attention map, and outputs the classified feature quantity map as a classification result of the training medical image input to the attention branch network 1A2.

In a general attention branch network, a loss function Loss of the attention branch network is expressed by Equation (1) below. In Equation (1), $CrossEntropyLoss_{att}$ indicates a learning error of the attention branch, and $CrossEntropyLoss_{per}$ indicates a learning error of the perception branch.

That is, in the general attention branch network, the loss function Loss of the attention branch network is a sum of the learning error $CrossEntropyLoss_{att}$ of the attention branch and the learning error $CrossEntropyLoss_{per}$ of the perception branch.

$$Loss = \mathrm{CrossEntropyLoss_{att}} + \mathrm{CrossEntropyLoss_{per}} \ (1)$$

Through intensive research, the present inventors have found the followings. When the loss function Loss of the attention branch network expressed in Equation (1) is used as the loss function Loss of the attention branch network 1A2, the convolutional neural network 1A1 focuses on a region which is not required for classifying the presence or absence of the disease on the chest X-ray image. In contrast, the loss function Loss of the attention branch network 1A2 expressed in Equation (2) below is used as the loss function Loss of the attention branch network 1A2. In this manner, the convolutional neural network 1A1 no longer focuses on the region which is not required for classifying the presence or absence of a disease on the chest X-ray image.

Specifically, through the research, the present inventors focus on a fact that the attention map output from the attention branch 1A22 has information corresponding to a location inside the training medical image. Furthermore, the present inventors adds a regularization term (third term on a right side in Equation (2)) to the loss function Loss of the attention branch network 1A2 so that the convolutional neural network 1A1 focuses on a region of a lung field or a heart on the chest X-ray image, and so that a high penalty is imposed when the convolutional neural network 1A1 focuses on a region other than the lung field or the heart on the chest X-ray image. As a result, the convolutional neural network 1A1 no longer focuses on the region which is not required for classifying the presence or absence of the disease on the chest X-ray image.

$$Loss = \mathrm{CrossEntropyLoss_{att}} + \mathrm{CrossEntropyLoss_{per}} + \lambda \|M \circ W\|_{\mathrm{Fro}}^{2} \ (2)$$

That is, the loss function Loss of the attention branch network 1A2 expressed in Equation (2) is the sum of the learning error $CrossEntropyLoss_{att}$ of the attention branch 1A22, the learning error $CrossEntropy Loss_{per}$ of the perception branch 1A23, and the regularization term. Specifically, the regularization term is a Frobenius norm of the matrix obtained by a Hadamard product of the attention map M and the weight map W output from the attention branch 1A22 (product of the attention map M and the weight map W for each component).

In Equation (2), a regularization parameter $\lambda$ is a hyperparameter that needs tuning each time during learning.

A weight map W corresponds to a region (classification region) on which the convolutional neural network 1A1 needs to focus. That is, the weight map W corresponds to the classification region which is a region required for classifying the presence or absence of the disease on the training medical image input to the medical image diagnostics assistance device 1.

That is, in the example indicated in FIG. 2, the attention branch network 1A2 is provided with preliminary information indicating the classification region which is a region required for classifying the presence or absence of the disease on the training medical image input to the medical image diagnostics assistance device 1.

As a result, in the examples represented in FIGS. 1 and 2, the convolutional neural network 1A1 can be prevented from focusing on the region which is not required for classifying the presence or absence of the disease on the training medical image. That is, it is possible to suppress the possibility that the medical image diagnostics assistance device 1 loses reliability from medical specialists since the convolutional neural network 1A1 focuses on the region which is not required for classifying the presence or absence of the disease on the training medical image.

In the examples represented in FIGS. 1 and 2, the classification model 1A does not carry out prognosis classification (that is, classifying the medical image into a medical image having good prognosis and a medical image having poor prognosis). Meanwhile, in another example, the classification model 1A may carry out the prognosis classification. In these examples, the training medical image for which the presence or absence of the disease is previously known and information on prognosis is previously known is used as the supervised data.

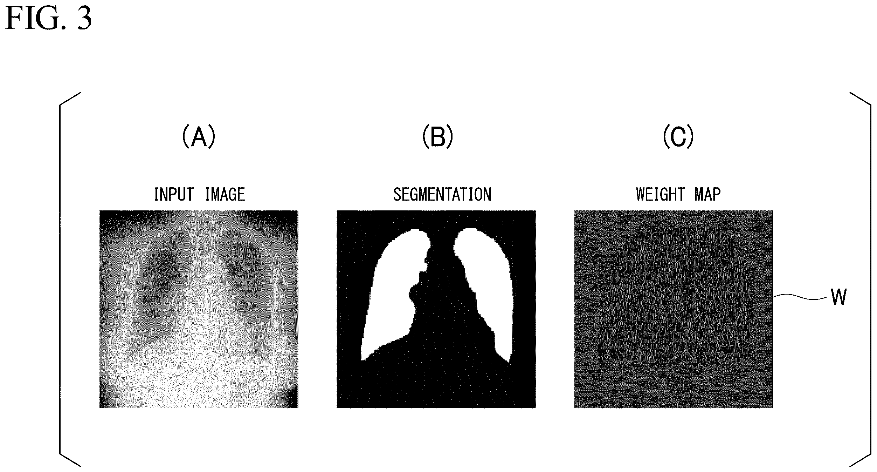

FIG. 3 is a diagram showing a method for preparing the weight map W in a first example of the medical image diagnostics assistance device 1 of the first embodiment. Specifically, FIG. 3(A) represents an example of the training medical image (input image) input to the medical image diagnostics assistance device 1. FIG. 3(B) represents a segmentation image a portion (lung field) of the region (classification region) required for classifying the presence or absence of the disease on the training medical image (chest X-ray image) represented in FIG. 3(A). FIG. 3(C) represents the weight map W prepared from the segmentation image represented in FIG. 3(B).

In the example indicated in FIG. 3, the weight map W represented in FIG. 3(C) is prepared by carrying out convex hull processing on the segmentation image (refer to FIG. 3(B)) of the first portion (specifically, the lung field) which is a portion of the classification region (specifically, the region required for classifying the presence or absence of the disease on the chest X-ray image represented in FIG. 3(A). For example, the weight map W represented in FIG. 3(C) is prepared by administrators (for example, medical specialists) of the medical image diagnostics assistance device 1. In a stage where the supervised learning of the classification model 1A is carried out by the learning unit 12, the attention branch network 1A2 of the medical image diagnostics assistance device 1 receives an input of the weight map W, and the weight map W is used to obtain the regularization term (that is, the weight map W is input to the attention branch network 1A2, and is used to obtain the regularization term).

In another example, the medical image diagnostics assistance device 1 may have a function for preparing the weight map W represented in FIG. 3(C).

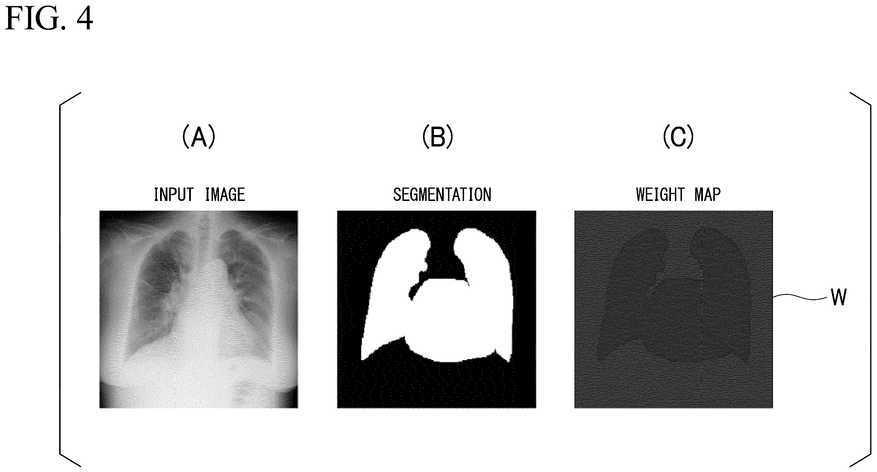

FIG. 4 is a diagram showing a method for preparing the weight map W in a second example of the medical image diagnostics assistance device 1 of the first embodiment. Specifically, FIG. 4(A) represents an example of a training medical image (input image) input to the medical image diagnostics assistance device 1. FIG. 4(B) represents a combination of the segmentation image of a portion (lung field) of the region (classification region) required for classifying the presence or absence of the disease and the segmentation image of another portion (heart) on the training medical image (chest X-ray image) represented in FIG. 4(A). FIG. 4(C) represents the weight map W prepared from the combination of the segmentation image of the lung field represented in FIG. 4(B) and the segmentation image of the heart.

In the example indicated in FIG. 4, the weight map W represented in FIG. 4(C) is prepared by generating the combination (refer to FIG. 4(B)) of the segmentation image of the first portion (specifically, the lung field) which is a portion of the classification region (specifically, a region required for classifying the presence or absence of the disease on the chest X-ray image represented in FIG. 4(A)) and the segmentation image of the second portion (specifically, the heart) which is another portion of the classification region. In a stage where the supervised learning of the classification model 1A is carried out by the learning unit 12, the attention branch network 1A2 of the medical image diagnostics assistance device 1 receives an input of the weight map W, and the weight map W is used to obtain the regularization term (that is, the weight map W is input to the attention branch network 1A2, and is used to obtain the regularization term).

In another example, the medical image diagnostics assistance device 1 may have a function for preparing the weight map W represented in FIG. 4(C).

Specifically, through the research, the present inventors use U-Net to generate the segmentation image (segmentation image of the lung field) represented in FIG. 3(B) and the segmentation image (combination of the segmentation image of the lung field and the segmentation image of the heart) represented in FIG. 4(B).

The U-Net is described in the following document.

O. Ronneberger, P. Fischer, and T. Brox, "U-net: Convolutional networks for biomedical image segmentation," Lect. Notes Comput. Sci. (including Subser. Lect. Notes Artif. Intell. Lect. Notes Bioinformatics), vol. 9351, pp. 234-241, 2015, doi: 10.1007/978-3-319-24574-4_28.

In addition, through the research, the present inventors use an example of a chest X-ray image 704 of Montgomery Country-Chest X-ray database for the supervised data of the lung field. The example of the chest X-ray image 704 of the Montgomery Country-Chest X-ray database is described in the following two documents.

S. Candemir et al., "Lung segmentation in chest radiographs using anatomical atlases with nonrigid registration," IEEE Trans. Med. Imaging, vol. 33, no. 2, pp. 577-590, 2014, doi: 10.1109/TMI.2013.2290491.

S. Jaeger et al., "Automatic Tuberculosis Screening Using Chest Radiographs," IEEE Trans. Med. Imaging, vol. 33, no. 2, pp. 233-245, Feb. 2014, doi: 10.1109/TMI.2013.2284099.

In addition, through the research, the present inventors use an example of a chest X-ray image 247 of JSRT for the supervised data of the heart. The example of the chest X-ray image 247 of JSRT is described in the following two documents.

P. (Project leader) T. U. of C. Junji Shiraishi et al., "Standard Digital Image Database:Chest Lung Nodules and Non-Nodules Created by the Japanese Society of Radiological Technology (JSRT) In cooperation with the Japanese Radiological Society (JRS)," 1997.

B. van Ginneken, M. B. Stegmann, and M. Loog, "Segmentation of anatomical structures in chest radiographs using supervised methods: A comparative study on a public database," Med. Image Anal., vol. 10, no. 1, pp. 19-40, 2006, doi: 10.1016/j.media.2005.02.002.

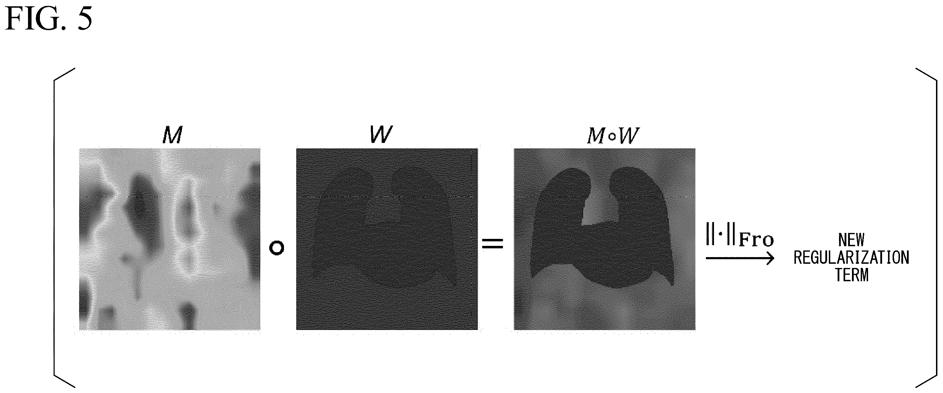

FIG. 5 is a diagram showing a calculation process of the regularization term in the medical image diagnostics assistance device 1 of the first embodiment.

In the medical image diagnostics assistance device 1 according to the first embodiment, as expressed in Equation (2) above and as represented in FIG. 5, the regularization term is calculated as the Frobenius norm of the matrix obtained by the Hadamard product of the attention map M and the weight map W (product of the attention map M and the weight map W for each component).

<Used data>

The present inventors use an example of a chest X-ray image 3032 captured at Teikyo University Hospital, in a research for verifying the medical image diagnostics assistance device 1 of the first embodiment. As a data configuration, a normal example (that is, an imaging subject of the chest X-ray image has no disease inside the chest X-ray image) includes an example 2002, and an abnormal example (that is, an imaging subject of the chest X-ray image has the disease inside the chest X-ray image) includes an example 1030. Some abnormal examples include an image captured when an imaging posture is a sitting position or a lying posture. As for an image size, the chest X-ray image is re-sized to 224×224 according to a recommended input size of the classification model.

<Classification Performance>

In the research for verifying the medical image diagnostics assistance device 1 of the first embodiment, the present inventors have carried out learning and accuracy evaluation of a chest X-ray data set of Teikyo University by using 10-fold cross-validation of stratified sampling. The learning and the accuracy evaluation of the medical image diagnostics assistance device 1 according to the first embodiment are carried out by constructing the attention branch network based on three typical CNNs (VGG16, ResNet50, and DenseNet121) as a classification model. The average and the standard deviation of Accuracy (Balanced Accuracy), Sensitivity, Specificity, and Area Under the Curve (AUC) are expressed in Table 1 below. In Table 1, "Weightmap1" indicates the weight map W (refer to FIG. 3(C)) obtained by carrying out convex hull processing on the segmentation image of the lung field, and "Weightmap2" indicates the weight map W (refer to FIG. 4(C)) obtained by combining the segmentation image of the lung field and the segmentation image of the heart.

[Table 1]

|  | Accuracy [%] | Sensitivity [%] | Specificity [%] | AUC |
| --- | --- | --- | --- | --- |
| VGG16 | 93.71 ± 1.50 | 91.26 ± 3.04 | 96.15 ± 1.60 | 0.98 ± 0.01 |
| VGG16 + ABN | 93.43 ± 1.54 | 90.00 ± 2.82 | 96.85 ± 1.67 | 0.97 ± 0.01 |

(continued)

|  | Accuracy [%] | Sensitivity [%] | Specificity [%] | AUC |
|---|---|---|---|---|
| VGG16 + ABN + weightmap1 | 93.74 ± 1.61 | 90.87 ± 3.01 | 96.60 ± 1.04 | 0.98 ± 0.01 |
| VGG16 + ABN + weightmap2 | 93.38 ± 1.52 | 90.00 ± 2.98 | 96.75 ± 1.47 | 0.97 ± .01 |
| Resnet50 | 93.16 ± 1.49 | 89.51 ± 3.03 | 96.80 ± 1.19 | 0.97 ± 0.01 |
| Resnet50 + ABN | 93.54 ± 1.55 | 91.17 ± 2.86 | 95.90 ± 1.43 | 0.97 ± 0.01 |
| Resnet50 + ABN + weightmap1 | 93.62 ± 1.48 | 90.29 ± 3.19 | 96.95 ± 1.91 | 0.98 ± 0.01 |
| Resnet50 + ABN + weightmap2 | 93.44 ± 1.81 | 90.78 ± 2.89 | 96.11 ± 2.24 | 0.98 ± 0.01 |
| Densenet121 | 93.99 ± 1.22 | 91.07 ± 2.29 | 96.90 ± 0.89 | 0.98 ± 0.01 |
| Densenet121 + ABN | 93.59 ± 1.45 | 90.68 ± 2.05 | 96.50 ± 2.07 | 0.98 ± 0.01 |
| Densenet121 + ABN + weightmap1 | 93.50 ± 1.51 | 90.19 ± 2.80 | 96.80 ± 1.20 | 0.98 ± 0.01 |
| Densenet121 + ABN + weightmap2 | 93.64 ± 1.26 | 90.78 ± 3.08 | 96.50 ± 1.21 | 0.98 ± 0.01 |

Specifically, a row of "VGG16" in Table 1 indicates an evaluation result of a medical image diagnostics assistance device in a comparative example in which VGG16 is used as CNN and to which ABN is not applied. A row of "VGG16+ABN" in Table 1 indicates an evaluation result of the medical image diagnostics assistance device in a comparative example in which VGG16 is used as CNN, to which ABN is applied, and in which ABN is not provided with the preliminary information (weight map W). A row of "VGG16+ABN+weightmap1" in Table 1 indicates an evaluation result in a first example of the medical image diagnostics assistance device 1 of the first embodiment in which VGG16 is used as the convolutional neural network 1A1, and the attention branch network 1A2 is provided with the weight map W (preliminary information) represented in FIG. 3(C). A row of "VGG16+ABN+weightmap2" in Table 1 indicates an evaluation result in a second example of the medical image diagnostics assistance device 1 of the first embodiment in which VGG16 is used as the convolutional neural network 1A1, and the attention branch network 1A2 is provided with the weight map W (preliminary information) represented in FIG. 4(C).

A row of "Resnet50" in Table 1 indicates an evaluation result of the medical image diagnostics assistance device in a comparative example in which ResNet50 is used as CNN and to which ABN is not applied. A row of "Resnet50+ABN" in Table 1 indicates an evaluation result of the medical image diagnostics assistance device in a comparative example in which ResNet 50 is used as CNN, to which ABN is applied, and in which ABN is not provided with the preliminary information (weight map W). A row of "Resnet50+ABN+weightmap1" in Table 1 indicates an evaluation result in the first example of the medical image diagnostics assistance device 1 of the first embodiment in which ResNet 50 is used as the convolutional neural network 1A1, and the attention branch network 1A2 is provided with the weight map W (preliminary information) represented in FIG. 3(C). A row of "Resnet50+ABN+weightmap2" in Table 1 indicates an evaluation result in the second example of the medical image diagnostics assistance device 1 of the first embodiment in which ResNet 50 is used as the convolutional neural network 1A1, and the attention branch network 1A2 is provided with the weight map W (preliminary information) represented in FIG. 4(C).

A row of "Densenet121" in Table 1 indicates an evaluation result of the medical image diagnostics assistance device in the comparative example in which DenseNet121 is used as CNN and to which ABN is not applied. A row of "Densenet121+ABN" in Table 1 indicates an evaluation result of the medical image diagnostics assistance device in the comparative example in which DenseNet121 is used as CNN, to which ABN is applied, and in which ABN is not provided with the preliminary information (weight map W). A row of "Densenet121+ABN+weightmap1" in Table 1 indicates an evaluation result in the first example of the medical image diagnostics assistance device 1 of the first embodiment in which DenseNet 121 is used as the convolutional neural network 1A1, and the attention branch network 1A2 is provided with the weight map W (preliminary information) represented in FIG. 3(C). A row of "Densenet121+ABN+weightmap2" in Table 1 indicates an evaluation result in the second example of the medical image diagnostics assistance device 1 of the first embodiment in which DenseNet121 is used as the convolutional neural network 1A1, and the attention branch network 1A2 is provided with the weight map W (preliminary information) represented in FIG. 4(C).

Balanced Accuracy is used as an evaluation index to properly evaluate a deviation in the number of data items between classes. Balanced Accuracy is expressed by Equation (3) below. In Equation (3), TP is True Positive, TN is True Negative, FP is False Positive, and FN is False Negative.

$$Balanced\ Accuracy = \frac{1}{2}\left(\frac{TP}{TP + FN} + \frac{TN}{FP + TN}\right) (3)$$

In any of the example indicated in the row of "VGG16+ABN+weightmap1" in Table 1, the example indicated in the row of "VGG16+ABN+weightmap2" in Table 1, the example indicated in the row of "Resnet50+ABN+weightmap1" in Table 1, the example indicated in the row of "Resnet50+ABN+weightmap2" in Table 1, the example indicated in the row of "Densenet121+ABN+weightmap1" in Table 1, and the example indicated in the row of "Densenet121+ABN+weightmap2" in Table 1, degraded performance due to addition of the regularization term of the medical image diagnostics assistance device 1 according to the first embodiment is not observed.

In addition, in the research for the learning and the accuracy evaluation of the medical image diagnostics assistance device 1 according to the first embodiment, the present inventors use a large-scale chest X-ray data set NIH14 published by the National Institute of Health. As in a case of Table 1, the learning and the accuracy evaluation of the medical image diagnostics assistance device 1 of the first embodiment are carried out by constructing the attention branch network based on three typical CNNs (VGG16, ResNet 50, and DenseNet 121) as the classification model. The average and the standard deviation of Accuracy, Sensitivity, Specificity, and AUC are expressed in Table 2 below. In Table 2, "Weightmap1" indicates the weight map W (refer to FIG. 3(C)) obtained by carrying out convex hull processing on the segmentation image of the lung field, and "Weightmap2" indicates the weight map W (refer to FIG. 4(C)) obtained by combining the segmentation image the lung field and the segmentation image of the heart.

In NIH14, the presence or absence of 14 diseases (Atelectasis, Cardiomegaly, Consolidation, Edema, Effusion, Emphysema, Fibrosis, Hernia, Infiltration, Mass, Nodule, Pleural Thickening, Pneumonia, and Pneumothorax) per one image is assigned by [0, 1]. The result indicated in Table 2 is the average value of 14 diseases.

[Table 2]

| NIH | Accuracy | Sensitivity | Specificity | AUC |
| --- | --- | --- | --- | --- |
| VGG16 | 0.68507 | 0.53562 | 0.83453 | 0.76588 |
| VGG16 + ABN | 0.65556 | 0.46155 | 0.84966 | 0.72754 |
| VGG16 + ABN + weightmap1 | 0.66107 | 0.42395 | 0.89819 | 0.76200 |
| VGG16 + ABN + weightmap2 | 0.67090 | 0.44139 | 0.9004 | 0.77126 |
| Resnet50 | 0.70791 | 0.62132 | 0.79449 | 0.78609 |
| Resnet50 + ABN | 0.71563 | 0.65516 | 0.7761 | 0.78819 |
| Resnet50 + ABN + weightmap1 | 0.71376 | 0.64286 | 0.78466 | 0.78746 |
| Resnet50 + ABN + weightmap2 | 0.71304 | 0.65263 | 0.77345 | 0.78598 |
| Densenet121 | 0.70438 | 0.61209 | 0.79667 | 0.78178 |
| Densenet121 + ABN | 0.71808 | 0.67070 | 0.76546 | 0.79249 |
| Densenet121 + ABN + weightmap1 | 0.71419 | 0.71498 | 0.71339 | 0.78946 |
| Densenet121 + ABN + weightmap2 | 0.71389 | 0.67858 | 0.7492 | 0.79011 |

Specifically, the row of "VGG16" in Table 2 indicates an evaluation result of the medical image diagnostics assistance device in the comparative example in which VGG16 is used as CNN and to which ABN is not applied. The row of "VGG16+ABN" in Table 2 indicates an evaluation result of the medical image diagnostics assistance device in the comparative example in which VGG16 is used as CNN, to which ABN is applied, and in which ABN is not provided with the preliminary information (weight map W). The row of "VGG16+ABN+weightmap1" in Table 2 indicates an evaluation result in the first example of the medical image diagnostics assistance device 1 of the first embodiment in which VGG16 is used as the convolutional neural network 1A1, and the attention branch network 1A2 is provided with the weight map W (preliminary information) represented in FIG. 3(C). The row of "VGG16+ABN+weightmap2" in Table 2 indicates an evaluation result in the second example of the medical image diagnostics assistance device 1 of the first embodiment in which VGG16 is used as the convolutional neural network 1A1, and the attention branch network 1A2 is provided with the weight map W (preliminary information) represented in FIG. 4(C).

The row of "Resnet50" in Table 2 indicates an evaluation result of the medical image diagnostics assistance device in the comparative example in which ResNet50 is used as CNN and to which ABN is not applied. The row of "Resnet50+ABN" in Table 2 indicates an evaluation result of the medical image diagnostics assistance device in the comparative example in which ResNet50 is used as CNN, to which ABN is applied, and in which ABN is not provided with the preliminary information (weight map W). The row of "Resnet50+ABN+weightmap1" in Table 2 indicates an evaluation result in the first example of the medical image diagnostics assistance device 1 of the first embodiment in which ResNet 50 is used as the convolutional neural network 1A1, and the attention branch network 1A2 is provided with the weight map W (preliminary information) represented in FIG. 3(C). The row of "Resnet50+ABN+weightmap2" in Table 2 indicates evaluation result in the second example of the medical image diagnostics assistance device 1 of the first embodiment in which ResNet 50 is used as the convolutional neural network 1A1, and the attention branch network 1A2 is provided with the weight map W (preliminary information) represented in FIG. 4(C).

The row of "Densenet121" in Table 2 indicates an evaluation result of the medical image diagnostics assistance device in the comparative example in which DenseNet121 is used as CNN and to which ABN is not applied. The row of "Densenet121+ABN" in Table 2 indicates an evaluation result of the medical image diagnostics assistance device in the comparative example in which DenseNet121 is used as CNN, to which ABN is applied, in which ABN is not provided with the preliminary information (weight map W). The row of "Densenet121+ABN+weightmap1" in Table 2 indicates an evaluation result in the first example of the medical image diagnostics assistance device 1 of the first embodiment in which DenseNet 121 is used as the convolutional neural network 1A1, and the attention branch network 1A2 is provided with the weight map W (preliminary information) represented in FIG. 3(C). The row of "Densenet121+ABN+weightmap2" in Table 2 indicates an evaluation result in the second example of the medical image diagnostics assistance device 1 of the first embodiment in which DenseNet121 is used as the convolutional neural network 1A1, and the attention branch network 1A2 is provided with the weight map W (preliminary information) represented in FIG. 4(C).

In any of the example indicated in the row of "VGG16+ABN+weightmap1" in Table 2, the example indicated in the row of "VGG16+ABN+weightmap2" in Table 2, the example indicated in the row of "Resnet50+ABN+weightmap1" in Table 2, the example indicated in the row of "Resnet50+ABN+weightmap2" in Table 2, the example indicated in the row of "Densenet121+ABN+weightmap1" in Table 2, and the example indicated in the row of "Densenet121+ABN+weightmap2" in Table 2, degraded performance due to addition of the regularization term of the medical image diagnostics assistance device 1 according to the first embodiment is not observed.

<Visualization of Determination Reason>

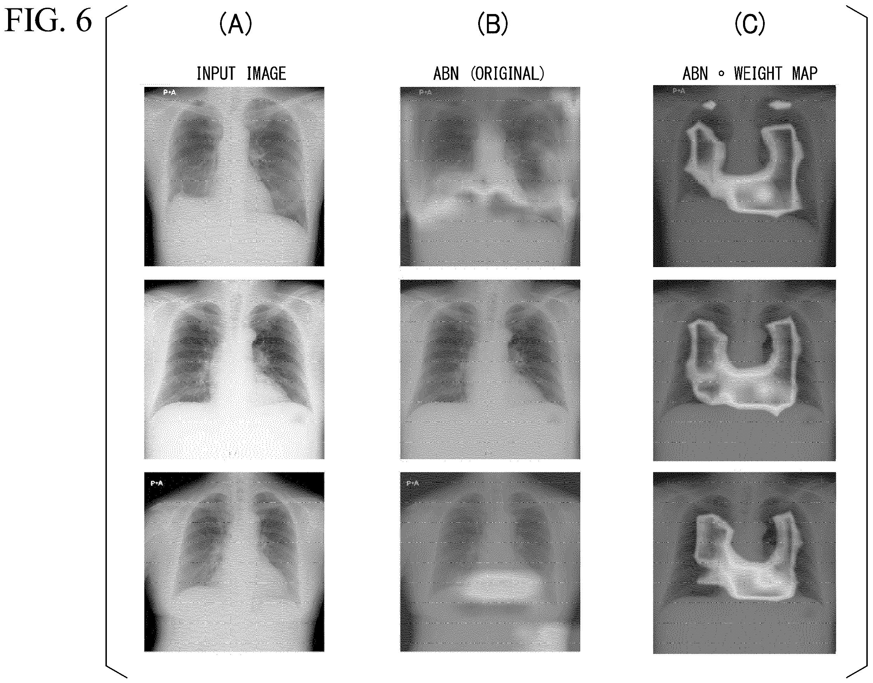

FIG. 6 is a diagram showing an interest region of the convolutional neural network 1A1 of the medical image diagnostics assistance device 1 of the first embodiment. Specifically, FIG. 6(A) represents the medical image (input image) input to the medical image diagnostics assistance device 1 of the first embodiment and the medical image diagnostics assistance device in the comparative example in which ABN is not provided with the preliminary information (weight map). FIG. 6(B) represents the interest region of CNN of the medical image diagnostics assistance device in the comparative example in which ABN is not provided with the preliminary information (weight map W). FIG. 6(C) represents the interest region of the convolutional neural network 1A1 of the medical image diagnostics assistance device 1 of the first embodiment in which the attention branch network 1A2 is provided with the preliminary information (weight map (specifically, the weight map W obtained by combining the segmentation image of the lung field and the segmentation image of the heart)).

In the example represented in FIG. 6, as the training medical image (input image) (refer to FIG. 6(A)) input to the medical image diagnostics assistance device 1 of the first embodiment and the medical image diagnostics assistance device in the comparative example in which ABN is not provided with the preliminary information (weight map), the chest X-ray data set of Teikyo University is used.

In the medical image diagnostics assistance device in the comparative example in which ABN is not provided with the preliminary information (weight map), as represented in FIG. 6(B), the attention map of ABN indicates that CNN focuses on the entire input image. Specifically, in the uppermost example in FIG. 6(B), the attention map of ABN indicates that CNN strongly focuses on a lower portion (portion including the lung field and the heart) on the input image, compared to an upper portion (portion which does not include the lung field and the heart) on the input image. In the second example from above in FIG. 6(B), the attention map of ABN indicates that CNN also focuses on a portion which does not need to be focused (portion which does not include the lung field and the heart) on the input image as equal as a portion which needs to be focused (portion including the lung field and the heart) on the input image. In the lowermost example of FIG. 6(B), the attention map of ABN indicates that CNN does not focus on a portion of the heart on the input image.

In contrast, in the medical image diagnostics assistance device 1 of the first embodiment in which the attention branch network 1A2 is provided with the preliminary information (weight map W), as represented in FIG. 6(C), the attention map of the attention branch network 1A2 indicates that the convolutional neural network 1A1 focuses on the inside of the lung field on the input image.

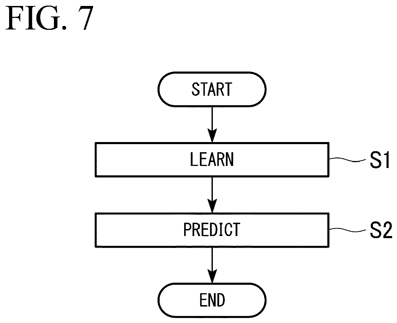

FIG. 7 is a flowchart showing an example of a process carried out in the medical image diagnostics assistance device 1 of the first embodiment.

In the example indicated in FIG. 7, in Step S1, the learning unit 12 carries out the supervised learning of the classification model 1A constructed by the convolutional neural network 1A1 and the attention branch network 1A2 that visualizes the interest region of the convolutional neural network 1A1. In the supervised learning carried out in Step S1, the training medical image for which the presence or absence of the disease is previously known is used as the supervised data. In Step S1, the attention branch network 1A2 is provided with information indicating the classification region (weight map W) which is a region required for classifying the presence or absence of the disease on the training medical image.

Next, in Step S2, the prediction unit 11 classifies the presence or absence of the disease from the medical image input to the medical image diagnostics assistance device 1 by using the trained classification model 1A.

In the example indicated in FIG. 7, the attention branch network 1A2 in Step S1 is provided with the preliminary information indicating the weight map W. Meanwhile, in another example, not only in Step S1 but also in Step S2, the attention branch network 1A2 may be provided with the preliminary information indicating the weight map W.

[Second Embodiment]

Hereinafter, a second embodiment of the medical image diagnostics assistance device, the medical image diagnostics assistance method, and the program of the present invention will be described.

The medical image diagnostics assistance device 1 of the second embodiment is configured as in the above-described medical image diagnostics assistance device 1 of the first embodiment, except for points to be described later. Therefore, according to the medical image diagnostics assistance device 1 of the second embodiment, the same advantageous effects as those of the medical image diagnostics assistance device 1 of the first embodiment can be achieved, except for points to be described later.

The medical image diagnostics assistance device 1 of the second embodiment is configured as in the medical image diagnostics assistance device 1 of the first embodiment represented in FIG. 1. That is, the medical image diagnostics assistance device 1 of the second embodiment includes the prediction unit 11, the learning unit 12, and the classification model 1A. The classification model 1A is constructed by the convolutional neural network 1A1 and the attention branch network 1A2 that visualizes the interest region of the convolutional neural network 1A1.

The attention branch network 1A2 of the medical image diagnostics assistance device 1 of the second embodiment is configured as in the attention branch network 1A2 of the medical image diagnostics assistance device 1 of the first embodiment represented in FIG. 2.

That is, for example, the attention branch network 1A2 of the medical image diagnostics assistance device 1 of the second embodiment includes the feature extractor 1A21 configured to generate the feature quantity map by extracting the feature quantity required for classifying the training medical image in a stage where the supervised learning of the classification model 1A is carried out by the learning unit 12, the attention branch 1A22, and the perception branch 1A23.

Specifically, in the medical image diagnostics assistance device 1 of the second embodiment, an output from the perception branch 1A23 is visualized by applying Grad-CAM to an output from the perception branch 1A23.

In the medical image diagnostics assistance device 1 of the second embodiment, as in the medical image diagnostics assistance device 1 of the first embodiment, the loss function Loss of the attention branch network 1A2 is the sum of the learning error CrossEntropyLoss$_{att}$ of the attention branch 1A22, the learning error CrossEntropyLoss$_{per}$ of the perception branch 1A23, and the regularization term.

In the medical image diagnostics assistance device 1 of the second embodiment, as in the medical image diagnostics assistance device 1 of the first embodiment, the regularization term is the Frobenius norm of the matrix obtained by the Hadamard product of the attention map M and the weight map W output from the attention branch 1A22.

In the medical image diagnostics assistance device 1 of the second embodiment, as in the medical image diagnostics assistance device 1 of the first embodiment, the weight map W corresponds to the region (classification region) on which the convolutional neural network 1A1 needs to focus. That is, the weight map W corresponds to the classification region which is a region required for classifying the presence or absence of the disease on the training medical image input to the medical image diagnostics assistance device 1.

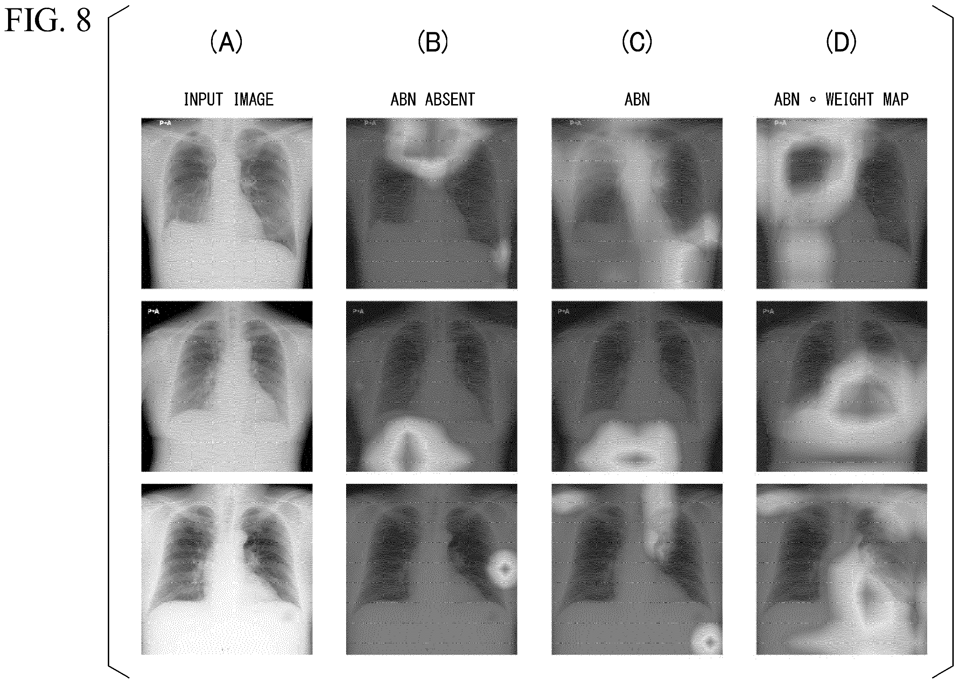

FIG. 8 is a diagram (Grad-CAM) showing the interest region of the convolutional neural network 1A1 of the medical image diagnostics assistance device 1 of the second embodiment. Specifically, FIG. 8(A) represents the medical image (input image) input to the medical image diagnostics assistance device 1 of the second embodiment, the medical image diagnostics assistance device in the comparative example to which ABN is not applied, and the medical image diagnostics assistance device in the comparative example to which ABN is applied and in which ABN is not provided with the preliminary information (weight map). FIG. 8(B) represents the interest region of CNN of medical image diagnostics assistance device of a comparative example to which ABN is not applied. FIG. 8(C) represents the interest region of CNN of the medical image diagnostics assistance device in the comparative example to which ABN is applied and in which ABN is not provided with the preliminary information (weight map). FIG. 8(D) represents the interest region of the convolutional neural network 1A1 of the medical image diagnostics assistance device 1 of the second embodiment in which the attention branch network 1A2 is provided with the preliminary information (weight map W).

In the example represented in FIG. 8, as the medical image (input image) (refer to FIG. 8(A) input the medical image diagnostics assistance device 1 of the second embodiment, the medical image diagnostics assistance device in the comparative example to which ABN is not applied, and the medical image diagnostics assistance device in the comparative example to which ABN is applied and in which ABN is not provided with the preliminary information (weight map), the chest X-ray data set of Teikyo University is used.

In the medical image diagnostics assistance device 1 of the second embodiment in which the attention branch network 1A2 is provided with the preliminary information (weight map W) in a stage where the supervised learning of the classification model 1A is carried out by the learning unit 12, as represented in FIG. 8(D) (as in the medical image diagnostics assistance device 1 of the first embodiment represented in FIG. 6(C)), the attention map of the attention branch network 1A2 indicates that the convolutional neural network 1A1 focuses on the inside of the lung field on the input image.

<Application Example>

In the above-described examples, the medical image diagnostics assistance device 1 of the first and second embodiments is applied to the chest X-ray image. Meanwhile, in another example, the medical image diagnostics assistance device 1 of the first or second embodiment may be applied to an X-ray image other than the chest X-ray image.

In another example, the medical image diagnostics assistance device 1 of the first or second embodiment may be applied to an echo image. Specifically, the convolutional neural network 1A1 focuses on a wall of the heart. In this manner, the classification model 1A can classify the presence or absence of a myocardial infarction. In addition, the convolutional neural network 1A1 focuses on a mammary gland. In this manner, the classification model 1A can classify the presence or absence of a chest cancer.

In still another example, the medical image diagnostics assistance device 1 of the first or second embodiment may be applied to an MRI image. Specifically, the convolutional neural network 1A1 focuses only on an intracranial region. In this manner, the classification model 1A can classify the presence or absence of a cerebral infarction. In addition, the convolutional neural network 1A1 focuses only on a prostate region. In this manner, the classification model 1A can classify the presence or absence of a prostate cancer.

In still another example, the medical image diagnostics assistance device 1 of the first or second embodiment may be applied to a CT image. Specifically, the convolutional neural network 1A1 focuses only on a site of the lung field. In this manner, the classification model 1A can classify the presence or absence of a pneumonia, a node, and a tumor. In addition, the convolutional neural network 1A1 focuses on only the intracranial region. In this manner, the classification model 1A can classify the presence or absence of a cerebral hemorrhage.

In still another example, the medical image diagnostics assistance device 1 of the first or second embodiment may be applied to a fundus image. Specifically, the convolutional neural network 1A1 focuses on an optic nerve papilla. In this manner, the classification model 1A can classify the presence or absence of a glaucoma.

Hitherto, the embodiments of the present invention have been described in detail with reference to the drawings. A specific configuration is not limited to the embodiments, and can be appropriately modified within the scope not departing from the concept of the present invention. The configurations described above in each of the embodiments and each of the examples may be combined.

All or a part of the medical image diagnostics assistance device 1 in the above-described embodiment may be realized by dedicated hardware, or may be realized by a memory and a microprocessor.

All or a part of the medical image diagnostics assistance device 1 may include a memory and a central processing unit (CPU), and may realize a function thereof in such a manner that a program for realizing a function of each unit included in each system is loaded to and executed by the memory.

A program for realizing all or a part of the functions of the medical image diagnostics assistance device 1 may be recorded on a computer-readable recording medium, and the program recorded on the recording medium may be read and executed by a computer system to carry out processing of each unit. The "computer system" referred herein includes an OS and hardware such as a peripheral device. In addition, the "computer system" includes a homepage providing environment (or a display environment) when a WWW system is used.

In addition, the "computer-readable recording medium" refers to a portable medium such as a flexible disk, a magneto-optical disk, a ROM, and a CD-ROM, and a storage device such as a hard disk built in the computer system. Furthermore, the "computer-readable recording medium" includes a medium which dynamically holds the program for a short period of time as in a communication line in a case where the program is transmitted through a network such as the Internet or a communication line such as a telephone line, and a medium which holds the program for a certain period of time as in a volatile memory inside the computer system serving as a server or a client in that case. In addition, the above-described program may be provided to realize a part of the above-described functions, or may be realized by combining the above-described functions with the program previously recorded in the computer system.

[Reference Signs List]

1:          Medical image diagnostics assistance device
11:         Prediction unit
12:         Learning unit

1A: Classification model
1A1: Convolutional neural network
1A2: Attention branch network
1A21: Feature extractor
1A22: Attention branch
1A23: Perception branch

**Claims**

1. A medical image diagnostics assistance device for assisting diagnosis of a medical image, comprising:

   a classification model configured to classify at least presence or absence of a disease from the medical image;
   a prediction unit configured to carry out prediction using the classification model; and
   a learning unit configured to carry out supervised learning of the classification model before the classification model is used by the prediction unit,
   wherein in the supervised learning carried out by the learning unit, a training medical image for which at least the presence or absence of the disease is previously known is used as supervised data,
   the classification model is constructed by a convolutional neural network and an attention branch network that visualizes an interest region of the convolutional neural network, and
   in a stage where the supervised learning of the classification model is carried out by the learning unit, the attention branch network is provided with preliminary information indicating a classification region which is a region required for classifying the presence or absence of the disease on the training medical image.

2. The medical image diagnostics assistance device according to Claim 1,

   wherein the attention branch network includes
   a feature extractor configured to generate a feature quantity map by extracting a feature quantity required for classifying the medical image,
   an attention branch configured to generate an attention map using class activation mapping, and
   a perception branch,
   in the stage where the supervised learning of the classification model is carried out by the learning unit, the attention map generated by the attention branch is reflected in the feature quantity map generated by the feature extractor,
   the perception branch outputs the feature quantity map weighted by the attention map, as a classification result of the training medical image,
   a loss function of the attention branch network is a sum of a learning error of the attention branch, a learning error of the perception branch, and a regularization term,
   the regularization term is Frobenius norm of a matrix obtained by a Hadamard product of the attention map and a weight map, and
   the weight map corresponds to the classification region.

3. The medical image diagnostics assistance device according to Claim 2,
   wherein in the stage where the supervised learning of the classification model is carried out by the learning unit, the attention branch network receives the weight map prepared by carrying out convex hull processing on a segmentation image of a first portion which is a portion of the classification region.

4. The medical image diagnostics assistance device according to Claim 2,
   wherein in the stage where the supervised learning of the classification model is carried out by the learning unit, the attention branch network receives the weight map prepared by combining a segmentation image of a first portion which is a portion of the classification region and a segmentation image of a second portion which is another portion of the classification region.

5. The medical image diagnostics assistance device according to Claim 3 or 4,
   wherein the segmentation image of the first portion and/or a combination of the segmentation image of the first portion and a segmentation image of a second portion which is another portion of the classification region are/is generated by using U-Net.

6. The medical image diagnostics assistance device according to Claim 1,
   wherein any one of VGG16, ResNet50, and DenseNet121 is used as the convolutional neural network.

7. The medical image diagnostics assistance device according to Claim 2,
   wherein an output from the perception branch is visualized by applying Grad-CAM to the output from the perception branch.

8. A medical image diagnostics assistance method for assisting diagnosis of a medical image, comprising:

   a prediction step of carrying out prediction using a classification model configured to classify at least presence or absence of a disease from the medical image; and
   a learning step of carrying out supervised learning of the classification model before the prediction step is carried out,
   wherein in the supervised learning carried out in the learning step, a training medical image for which at least the presence or absence of the disease is previously known is used as supervised data,
   the classification model is constructed by a convolutional neural network and an attention branch network that visualizes an interest region of the convolutional neural network, and
   in the learning step, the attention branch network is provided with preliminary information indicating a classification region which is a region required for classifying the presence or absence of the disease on the training medical image.

9. A program that causes a computer to carry out steps comprising:

   a prediction step of carrying out prediction using a classification model configured to classify at least presence or absence of a disease from a medical image; and
   a learning step of carrying out supervised learning of the classification model before the prediction step is carried out,
   wherein in the supervised learning carried out in the learning step, a training medical image for which at least the presence or absence of the disease is previously known is used as supervised data,
   the classification model is constructed by a convolutional neural network and an attention branch network that visualizes an interest region of the convolutional neural network, and
   in the learning step, the attention branch network is provided with preliminary information indicating a classification regAion which is a region required for classifying the presence or absence of the disease on the training medical image.

## FIG. 1

EP 4 407 554 A1

FIG. 2

MEDICAL IMAGE → FEATURE EXTRACTOR (1A21) → FEATURE QUANTITY MAP

ATTENTION MAP

ATTENTION BRANCH (1A22) ← PRELIMINARY INFORMATION (WEIGHT MAP)

WEIGHED FEATURE QUANTITY MAP → PERCEPTION BRANCH (1A23) → CLASSIFICATION RESULT

1A2

EP 4 407 554 A1

FIG. 3

(A) INPUT IMAGE  (B) SEGMENTATION  (C) WEIGHT MAP

FIG. 4

(A) INPUT IMAGE

(B) SEGMENTATION

(C) WEIGHT MAP

W

FIG. 5

$$M \quad W \quad M \circ W$$

$$M \circ W \xrightarrow{\| \cdot \|_{\mathrm{Fro}}} \quad \text{NEW REGULARIZATION TERM}$$

EP 4 407 554 A1

FIG. 6

FIG. 7

FIG. 8

(A)      (B)      (C)      (D)

INPUT IMAGE      ABN ABSENT      ABN      ABN ∘ WEIGHT MAP

EP 4 407 554 A1

## INTERNATIONAL SEARCH REPORT

| International application No. |
|---|
| **PCT/JP2022/033653** |

### A. CLASSIFICATION OF SUBJECT MATTER

*G06T 7/00*(2017.01)i; *A61B 6/00*(2006.01)i; *A61B 6/03*(2006.01)i
FI: A61B6/00 350D; A61B6/00 350Z; A61B6/03 360J; A61B6/03 360Z; G06T7/00 612

According to International Patent Classification (IPC) or to both national classification and IPC

### B. FIELDS SEARCHED

Minimum documentation searched (classification system followed by classification symbols)

G06T7/00; G06N3/00; A61B6/00; A61B6/03

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); インターネット

### C. DOCUMENTS CONSIDERED TO BE RELEVANT

| Category* | Citation of document, with indication, where appropriate, of the relevant passages | Relevant to claim No. |
|---|---|---|
| Y | JP 6657480 B2 (JAPANESE FOUND FOR CANCER RES) 04 March 2020 (2020-03-04) paragraphs [0019]-[0055], [0060], etc., in particular, paragraphs [0019]-[0020], [0026], [0028]-[0031], [0048]-[0052], [0060], etc. | 1, 6, 8-9 |
| Y | JP 2021-22368 A (UNIV CHUBE) 18 February 2021 (2021-02-18) paragraphs [0002], [0004], [0016]-[0085] | 1, 6, 8-9 |
| A | JP 2020-119154 A (CANON KK) 06 August 2020 (2020-08-06) entire text, all drawings | 1-9 |
| A | US 2021/0150728 A1 (MODIFACE INC.) 20 May 2021 (2021-05-20) entire text, all drawings | 1-9 |
| A | US 2019/0362183 A1 (IDEMIA IDENTITY & SECURITY FRANCE) 28 November 2019 (2019-11-28) entire text, all drawings | 1-9 |

☑ Further documents are listed in the continuation of Box C.    ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **05 October 2022** | **18 October 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

International application No.

**PCT/JP2022/033653**

## C.    DOCUMENTS CONSIDERED TO BE RELEVANT

| Category* | Citation of document, with indication, where appropriate, of the relevant passages | Relevant to claim No. |
|---|---|---|
| A | TAHEREH, Hassanzadeh et al. Evolutionary Attention Network for Medical Image Segmentation. 2020 Digital Image Computing: Techniques and Applications (DICTA). 2020, pp. 1-8, [retrieval date: 05 October 2022], <DOI: 10.1109/DICTA51227.2020.9363425>, <available at https://ieeexplore.ieee.org/document/9363425> entire text, all drawings | 1-9 |
| A | OLAF, Ronneberger et al. U-Net: Convolutional Networks for Biomedical Image Segmentation. Medical Image Computing and Computer-Assisted Intervention - MICCAI 2015. 2015, pp. 234-241, [retrieval date: 05 October 2022], <available at https://link.springer.com/chapter/10.1007/978-3-319-24574-4_28> entire text, all drawings | 1-9 |
| A | FUKUI, Hiroshi et al. Attention Branch Network: Learning of Attention Mechanism for Visual Explanation. 2019 IEEE/CVF Conference on Computer Vision and Pattern Recognition (CVPR). 2019, pp. 10697-10706, [retrieval date: 05 October 2022], <DOI: 10.1109/CVPR.2019.01096>, <available at https://ieeexplore.ieee.org/document/8953929> entire text, all drawings | 1-9 |
| A | JP 6-251038 A (TOSHIBA CORP) 09 September 1994 (1994-09-09) entire text, all drawings | 1-9 |
| A | US 2021/0090247 A1 (SAMSUNG SDS CO., LTD.) 25 March 2021 (2021-03-25) entire text, all drawings | 1-9 |
| A | JP 2018-117883 A (UNIV OSAKA) 02 August 2018 (2018-08-02) entire text, all drawings | 1-9 |
| A | US 10430946 B1 (INCEPTION INSTITUTE OF ARTIFICIAL INTELLIGENCE, LTD.) 01 October 2019 (2019-10-01) entire text, all drawings | 1-9 |
| P, X | WO 2022/172817 A1 (FUJIFILM CORP  ) 18 August 2022 (2022-08-18) paragraphs [0011], [0036]-[0063] | 1, 6, 8-9 |

Form PCT/ISA/210 (second sheet) (January 2015)

**INTERNATIONAL SEARCH REPORT**
Information on patent family members

International application No.

**PCT/JP2022/033653**

| Patent document cited in search report | | | Publication date (day/month/year) | Patent family member(s) | | | Publication date (day/month/year) |
|---|---|---|---|---|---|---|---|
| JP | 6657480 | B2 | 04 March 2020 | US 2020/0337537 A1 paragraphs [0069]-[0110], [0118], etc., in particular, paragraphs [0069]-[0071], [0077], [0080]-[0084], [0102]-[0107], [0118], etc. WO 2019/088121 A1 EP 3705025 A1 KR 10-2020-0106028 A CN 111655116 A | | | |
| JP | 2021-22368 | A | 18 February 2021 | (Family: none) | | | |
| JP | 2020-119154 | A | 06 August 2020 | (Family: none) | | | |
| US | 2021/0150728 | A1 | 20 May 2021 | WO 2021/092687 A1 | | | |
| US | 2019/0362183 | A1 | 28 November 2019 | EP 3572976 A1 | | | |
| JP | 6-251038 | A | 09 September 1994 | US 5807256 A entire text, all drawings EP 616290 A2 | | | |
| US | 2021/0090247 | A1 | 25 March 2021 | KR 10-2021-0035381 A | | | |
| JP | 2018-117883 | A | 02 August 2018 | (Family: none) | | | |
| US | 10430946 | B1 | 01 October 2019 | WO 2020/183230 A1 | | | |
| WO | 2022/172817 | A1 | 18 August 2022 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2021153427 A
- JP 6657480 B

### Non-patent literature cited in the description

- **H. FUKUI ; T. HIRAKAWA ; T. YAMASHITA ; H. FU-JIYOSHI.** Attention branch network: Learning of attention mechanism for visual explanation. *Proc. IEEE Comput. Soc. Conf. Comput. Vis. Pattern Recognit.,* 2019, vol. 2019-June, 10697-10706
- **O. RONNEBERGER ; P. FISCHER ; T. BROX.** U-net: Convolutional networks for biomedical image segmentation. *Lect. Notes Comput. Sci. (including Subser. Lect. Notes Artif. Intell. Lect. Notes Bioinformatics),* 2015, vol. 9351, 234-241
- **S. CANDEMIR et al.** Lung segmentation in chest radiographs using anatomical atlases with nonrigid registration. *IEEE Trans. Med. Imaging,* 2014, vol. 33 (2), 577-590
- **S. JAEGER et al.** Automatic Tuberculosis Screening Using Chest Radiographs. *IEEE Trans. Med. Imaging,* February 2014, vol. 33 (2), 233-245
- **C. JUNJI SHIRAISHI et al.** Standard Digital Image Database:Chest Lung Nodules and Non-Nodules Created by the Japanese Society of Radiological Technology (JSRT) In cooperation with the Japanese Radiological Society (JRS). *P. (Project leader) T. U.,* 1997
- **B. VAN GINNEKEN ; M. B. STEGMANN ; M. LOOG.** Segmentation of anatomical structures in chest radiographs using supervised methods: A comparative study on a public database. *Med. Image Anal.,* 2006, vol. 10 (1), 19-40